# EUROPEAN PATENT SPECIFICATION

(11) **EP 2 254 626 B1**
(45) Date of publication and mention of the grant of the patent: **03.07.2013**
(21) Application number: 09709168.0
(22) Date of filing: 22.01.2009
(51) Int. Cl.: A61M 5/31

(54) **SYRINGE WITH PISTON SEAL**
SPRITZE MIT KOLBENDICHTUNG
SERINGUE MUNIE D UN JOINT DE PISTON

(30) Priority: 05.02.2008 NL 2001258
(43) Date of publication of application: 01.12.2010
(73) Proprietor: Helvoet Rubber & Plastic Technologies B.v., 3223 EV Hellevoetsluis (NL)
(72) Inventor: VAN DER MOLEN, Peter-Jan, NL-2496 PP Den Haag (NL); VAN VESSEM, Louis, NL-3223 GG Hellevoetsluis (NL)
(74) Representative: Riemens, Roelof Harm
(86) International application number: PCT/NL2009/000012
(87) International publication number: WO 2009/099321

(56) References cited:
- WO-A-2005/089831
- US-A- 4 367 738
- US-A- 5 578 015

## Description

The invention relates to a syringe with a seal that is also referred to as a tamper evident that discernibly seals a piston body with regard to a cylindrical part of a syringe body of the syringe.

Tamper evident syringes are already known in a number of variants. For example, US 6,565,529 B1 shows a syringe with a piston body that has been equipped with a stop disc on its piston rod. The stop disc lies pointing backwards against a limiting flange of the syringe body that bends inwardly. The stop disc and limiting flange that stop each other together form a tamper evident. The tamper evident ensures that a sharp needle cannot be stuck along the piston body into the syringe to subsequently illicitly and unnoticed mess with the contents. The tamper evident also ensures that nobody can pull the piston body illicitly and unnoticed from the syringe body. Both operations will lead to visible damage to the limiting flange.

A negative aspect related to this known syringe is that the manufacture and the assembling thereof leave a lot to be desired. The syringe can also be improved with regard to when it is being used. Another negative aspect is that first a number of other operations must be performed before the tamper evident can be made operational. For example, first the different parts of the syringe must be produced, then the syringe must be filled with an injection medium and, thereafter, the piston body must be placed in the cylindrical part of the syringe body. Only then can the tamper evident be activated. This takes place here through a thermal deformation process of the limiting flange to ensure that it is bent inwardly in such a way that this starts to form a limit for the stop disc. This critical sequence of acting limits the production, the assembly and the filling process which, as it where, are intermingled. This also entails the risk that the injection medium and/or other parts of the syringe will be negatively influenced by the thermal deformation process as a result of the high temperatures that are required for this. It is also difficult in practice to continuously impose a uniform deformation on the limiting flange, which may not be beneficial for the end user and/or the patient, for example, if the limiting flange deforms to such an extent internally that it comes to lie too tightly against the piston rod and hinders, due to this, the free internal sliding movement thereof.

US 4,367,738 shows a syringe with a piston body comprising outwardly projecting spikes. A cylinder of the syringe comprises a restricted opening. During operation the spikes are slightly compressed to pass through this restricted opening. Withdrawal of the plunger assembly is not possible because the spikes will not pass backward through the restricted opening.

A disadvantage of this known syringe is that a piston head which is provided at the front end of the piston body is unable to pass through the restricted opening, because of which the restricted opening can only be made after the piston head is placed into the cylinder.

The present invention aims to at least partially overcome the aforementioned negative aspects and/or to provide a useable alternative. In particular, the invention aims to provide a reliable and easily to be produced syringe with seal.

This aim is achieved through a syringe in accordance with claim 1. With this the syringe body comprises a cylindrical part that has been equipped with a nozzle at its distal end. The syringe body is closed in a sealing manner on its proximal end by a piston body that can be slid in the cylindrical part. Further a piston seal has been provided that at least comprises one limiting section linked to the syringe body or integrally formed that protrudes inwardly in a radial direction. The piston body is provided with at least one stop section that is linked to this or has been integrally formed on it that protrudes outwardly in a radial direction and that is formed by a stop cam that can be inwardly compressed in the radial direction. The limiting section has radial internal dimensions that are greater than the radial external dimensions of the piston and/or the radial internal dimensions of the cylindrical part of the syringe body. The at least one stop cam has radial external dimensions that are greater than the radial internal dimensions of the limiting section. Furthermore the limiting section is constructed to become discernibly damaged, for example, by clearly breaking, if an attempt is made to pull the piston body from the syringe body. The at least one stop cam will automatically encounter the limiting section that is dimensioned as being smaller in the radial direction when such a pulling out movement occurs and, thus, automatically ensures that a resistance against pulling out is felt and that the limiting section becomes damaged visibly and/or audibly when the pulling-out force is greater. The stop cam and the limiting section together form a piston seal that can be used as a tamper evident. It is advantageously possible with regard to the piston seal in accordance with the invention to put the piston body in the syringe body while the limiting section of the piston seal is already in its operational position. Also it is possible to first place the piston body in the syringe body and only thereafter place the limiting section of the piston seal in its operational position. The piston has smaller radial dimensions and can, therefore, pass the limiting section. Further the stop cam has the freedom to inwardly compress in the radial direction and can, thus, also pass the limiting section. The manufacture and assembly of the syringe can be carried out quickly and easily due to this, including any filling with injection medium, while at the same time more freedom is obtained in the sequence of actions.

It is noted that WO 2005/089831 and US 5,578,015 show syringes with compressible stop fingers for limiting relative axial movements of cylinder and piston body parts. Those compressible stop fingers, however, do not form part of a tamper evident construction, only prevent an axial inward movement of the piston body, and merely are destined to prevent a premature inactivation of a needle of the syringe to a position in which it can no longer injure people.

By preference, the limiting section according to the present invention is provided as a separate breaking body that has been secured in the proximal end of the syringe body, for example, through a snap connection. The benefit of this is that it is no longer necessary to subject the syringe during assembly and filling to a thermal deformation process with the related high temperatures. Both the breaking body and other parts of the syringe can beneficially be manufactured from plastic, for example, in an injection moulding process.

In a further embodiment, the breaking body is built up from at least two ring-shaped segments that are interlinked through weakened wall sections (breaking walls). The ring-shaped segments can fully enclose the piston rod of the piston body together and, thus, can form a limit over the whole of the perimeter that will prevent, for example, that a sharp hypodermic needle can be stuck inwardly from the rear along the piston body in the syringe body to extract the injection medium from this.

In a variant, the breaking body is built up from a mounting part that is inseparably installed on the syringe body and a ring-shaped breaking-out segment linked to the mounting part through weakened wall sections (breaking walls). The ring-shaped breaking-out segment can then again fully enclose the piston rod and form a limit with regard to the whole of the perimeter.

Advantageously the at least one stop cam that can be inwardly compressed in the radial direction has been integrally formed on the piston body, in particular, on the piston rod thereof. The piston rod can, for example, be equipped with a hollow cylindrical part where a recess has been left free in the cylindrical perimeter wall in which the stop cam has the freedom of inwardly compressing. Furthermore the piston rod can be provided with a differently shaped cross section, for example, solidly and/or with wall sections that are cruciform, star-shaped or the like seen in cross section, in which the at least one compressable stop cam can then be integrated in one or more of such wall sections.

In a further embodiment, the nozzle comprises a separate needle unit that has been inserted into the syringe body from the proximal end before the piston body has been stuck therein. The needle unit can advantageously have been linked from the inside with the syringe body, for example, through a snap connection. Together with the forming of the limiting body that is not integral with the syringe body, this embodiment ensures that the needle unit and the breaking body can be inserted together into the syringe body with one continuous action and can be secured therein at their respective positions through, for example, the aforementioned snap connections.

The breaking body and the needle unit can, in the first instance, be interlinked during manufacture to great advantage. In particular, they can even be manufactured as an integral part with, for example, weakened wall sections as breaking walls in-between. During assembly, the weakened wall sections can be broken as a result of an axial insertion force that is exercised on the needle unit. This will take place automatically if the axial insertion force continues to exert pressure on the needle unit after the breaking body has reached its end position and there has been jammed within the syringe body. The needle unit will then be pressed in the direction of the distal end of the syringe body as a result of the continuous axial insertion force in one continuous movement and will there be jammed in the syringe body.

In a special embodiment, a nozzle seal has been provided as a tamper evident on the distal end besides the piston seal as tamper evident on the proximal end. The nozzle seal is also constructed to become discernibly damaged, for example, by clearly breaking, if an attempt is made to release the outflow opening of the nozzle. Thus, a syringe is created that can resist illicit use on all sides and/or will leave clear traces behind of this.

In a further embodiment, the nozzle seal has been provided as a protective cap that at least protects the outflow opening of the nozzle and that is integrally formed on the distal end of the syringe body as a breaking wall through weakened wall sections. This integral forming is advantageously possible in combination with the simple construction of the piston seal and/or owing to the needle unit that is provided as a separate component that can be fitted from the proximal end into the syringe body.

Preferably, the protective cap is provided in such a way that it lies against the outflow opening of the nozzle and/or the outflow opening of the needle unit, respectively in a sealing position in the non-broken position. This makes a separate sealing of the outflow opening superfluous and prevents unnecessary contamination of the outflow opening and unnecessary loss of injection medium if the protective cap is removed.

The integrally formed protective cap preferably is built up from at least two parts that are interlinked through weakened wall sections as breaking wall. This will give the user the option to adjust a required penetration depth of the nozzle and a needle of the needle unit, respectively, into a body part. By only removing the front part, the user will release a shorter longitudinal part of the nozzle and needle, respectively, than when he removes the entire protective cap.

Further preferred embodiments are defined in the subclaims.

The invention also relates to the use of the syringe for injecting an injection medium into animals, in particular, in a teat of an udder of a milking animal such as a cow or a goat.

The invention will be further explained based on the included drawings in which:
Figure 1 is a schematic view in perspective of an embodiment of a syringe in accordance with the invention before assembly;
Figure 2 is a partially exploded view in accordance with figure 1 with a needle unit and breaking body mounted in the syringe body;
Figure 3 is a view in accordance with figure 2 with the piston body mounted in the syringe body;
Figure 4 is a view in accordance with figure 2 with a broken and pulled out breaking body;
Figure 5 is an enlarged partial view with the piston body fully pressed in;
Figure 6 is an enlarged view of the assembly of needle unit with the breaking body from figure 1;
Figure 7 shows a variant with an alternative type of needle;
Figure 8 shows three steps of the removal of the nozzle seal;
Figure 9 shows a variant of figure 8 with a one-piece nozzle seal;
Figure 10a and 10b show a variant of figure 1, 3 with a piston body equipped with additional safety cams; and
Figure 11-16 are views of a variant in accordance with figure 1-6 with a cruciform piston rod and a breaking body with a mounting section and a ring-shaped breaking-out segment.

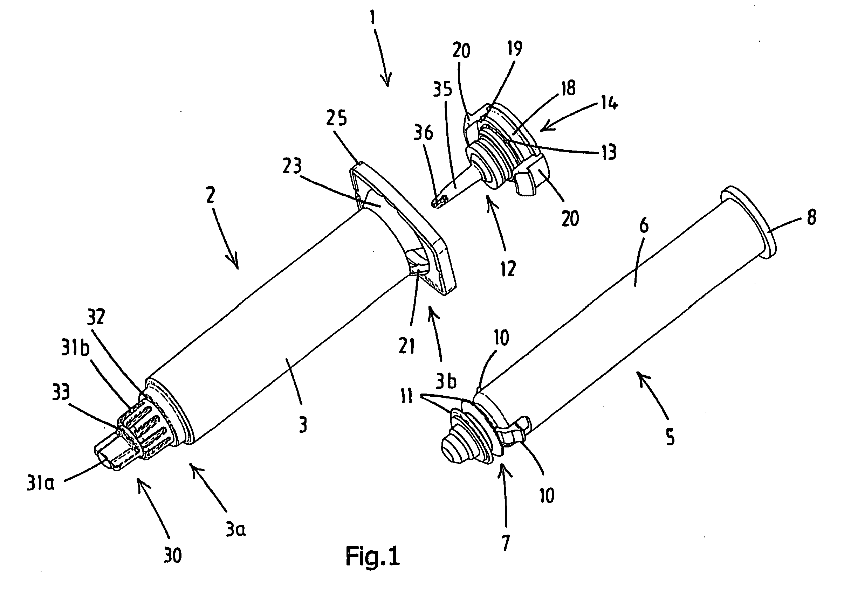

In figure 1 the syringe has been designated in its entirety with the reference number 1. The syringe 1 comprises a syringe body 2 with a cylindrical part 3 with an axial direction on which a distal end 3a and a proximal end 3b can be identified. The syringe 1 also comprises a piston body 5 with a piston rod 6 and a piston 7. The piston rod 6 changes into a thumb support 8 on its free end and is further equipped with a cylindrical wall section in which two integrally formed stop cams 10 with a diametrically opposed position have been provided. The stop cams 10 extend, as it where, in recesses in the cylindrical wall section and have, due to this, the freedom to inwardly compress in the radial direction. The piston 7 is also an integral part of the piston body 5 here and is equipped with two elastically deformable ring-shaped wall sections 11. The piston 7 has been dimensioned in such a way that it lies in a sealing position against the internal perimeter wall of the cylindrical part 3 with its wall sections 11 in a mounted position. The radial external dimensions of the piston 7 are mainly the same as the radial internal dimensions of the cylindrical part 3 for this.

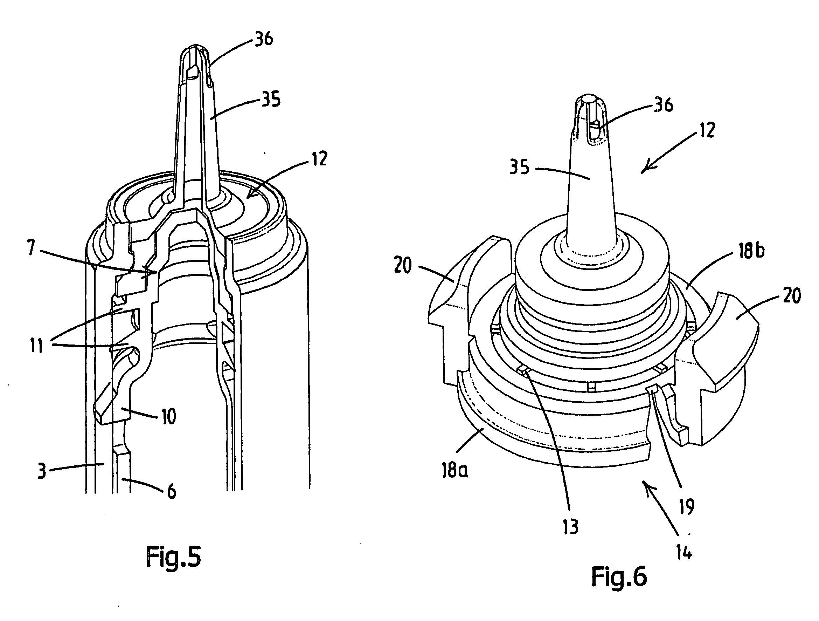

As a further part before assembly, the syringe 1 comprises an integrally formed assembly of a needle unit 12 that is connected to a breaking body 14 (also see Fig. 6) through weakened wall sections 13. This assembly is destined to be broken during the assembly as will be further discussed below.

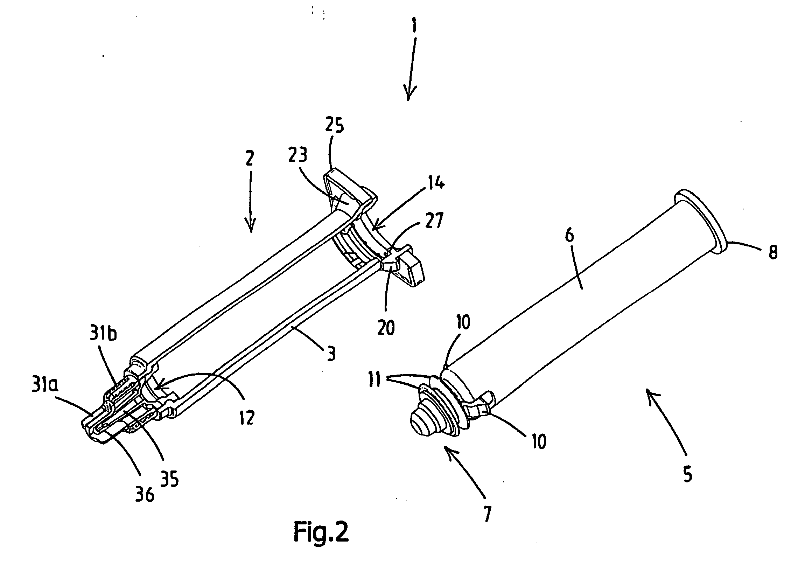

The needle unit 12 is destined to form a nozzle on the distal end 3a and should be inserted into the syringe body 2 from the proximal end 3b. At the location of the distal end 3a, the needle unit 12 can be permanently connected to the syringe body 2. This takes place through a snap connection with wall sections that complementarily grip into each other, in particular, a spring-groove connection with regard to the embodiment shown here (see fig. 2).

The breaking body 14 comprises two semicircular-shaped segments 18a, 18b that are interlinked through weakened wall sections 19. The breaking body 14 can be permanently connected to the proximal end 3b of the syringe body 2. In the shown embodiment, this connection is also formed through a snap connection, for which each segment 18a, 18b is equipped with a mounting boss 20 that can grip itself into a complementary recess 21 that is provided in a widened part 23 of the proximal end 3b of the syringe body 2 (see fig. 2). The widened part 23 changes into finger grips 25 for the user.

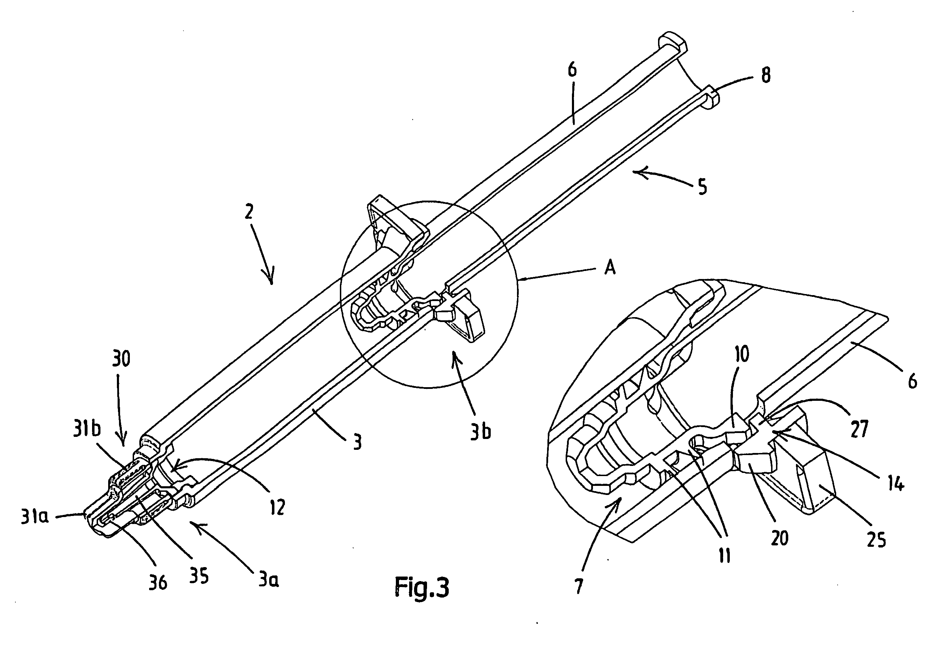

The breaking body 14 comprises an inwardly protruding limiting section 27 in the radial direction that extends closed in the circumferential direction. The limiting section 27 is executed with an internal diameter that is greater than the external diameter of the wall sections 11 of the piston 7 and the internal diameter of the cylindrical part 3, respectively. This ensures that the piston 7 can be stuck through the breaking body 14, in particular after the breaking body 14 has been separated of the needle unit 12 and has been mounted in the proximal end 3b of the syringe body 2. The stop cams 10, in turn, have, however, been executed with radial external dimensions that in the non-compressed position are, on the contrary, greater than the internal diameter of the limiting section 27. If the piston body 5 is stuck through the breaking body 14, the stop cams 10 will be made to inwardly compress in the radial direction as a result of their tapering fronts. Only in this compressed position can the stop cams 10 pass the limiting section 27 of the breaking body 14. When the stop cams 10 have passed the limiting section 27, they will again have the freedom to spring outwardly. From this moment on, the piston body 5 with its stop cams 10 in axial direction is limited by the limiting section 27 of the breaking body 14 (see fig. 3).

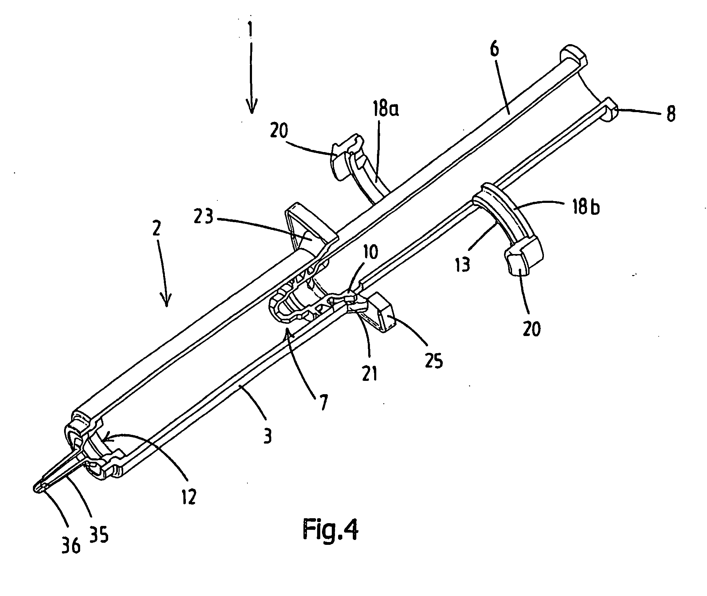

If an attempt is made to again pull the piston body 5 from the syringe body 2, this will immediately lead to the breaking body 14 blocking this. The breaking body 14 has been constructed in such a way that it can only again be removed from the syringe body 2 after it has been broken in two or more pieces. As a result of the acting interplay of forces between the stop cams 10 and the limiting section 27, the breaking body 14 will break when a sufficiently extensive pulling out force is exerted on the piston body 5 where the weakened wall sections 19 can be found. This will produce two independent segments 18a, 18b that will have the freedom to be released with their mounting boss 20 from the recess and then be pulled out from the syringe body 2 outwardly together with the piston body 5 (see fig. 4). A possibility is that the segments are released completely and another possibility is that the segments break off from each other when the piston is pulled out but that these distorted segments do remain affixed to the cylinder. This is advantageous because then no pieces will break loose. The breaking body 14, thus, forms a reliable piston seal together with the stop cams 10 that will make the illicit removal of the piston body 5 from the syringe body 2 visible.

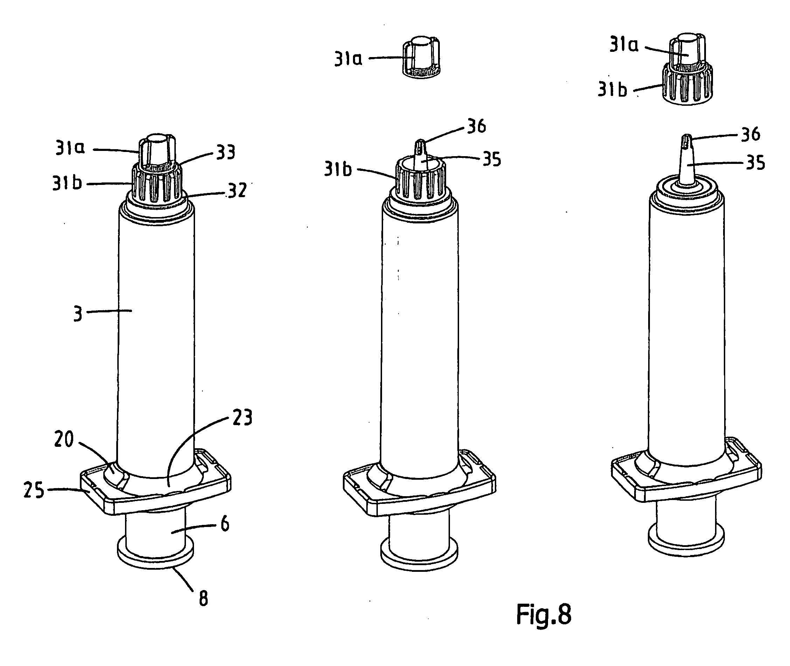

A seal is provided on the distal end 3a of the syringe body 2 of the nozzle formed by the needle unit 12. This nozzle seal 30 is here formed by a protective cap 31 that is integrally formed through a weakened perimeter wall section 32 on the syringe body 2. The protective cap 31 has here been provided as a two-piece section where both parts 31 a, 31 b are again interlinked through a weakened perimeter wall section 33. This ensures that only the front part 31 a can be removed or also the back part 31 b. As can be seen in Figure 8, this gives the user the option to release a longer or shorter section of the needle 35 of the needle unit 12 to introduce in a body section. Both parts of the protective cap 31 are equipped with such profiles that a user can have a good grip; here formed through ribs and wings, respectively. The front part 31 a has, furthermore, been executed with a smaller external diameter and, due to this, also with a smaller perimeter on the weakened wall section 33 than the back part 31 b with its weakened perimeter wall section 32. This ensures to advantage that when a turning force is applied on the front part 31 a, this front part 31 a will break away from the back part 31b at the location of its weakened perimeter wall section 33. Only if the user applies a turning force on the back part 31 b this will break away from the rest of the syringe body 2 at the location of its weakened perimeter wall section 32 can be found.

The protective cap 31 of the nozzle seal 30 has been executed in such a way that this rests in a sealing position with its front part 31 a against the outflow openings 36 of the needle 35 of the needle unit 12.

To great advantage, the shown syringe 1 comprises only three parts before assembly that can all be made from plastic, in particular, PE, PP and or TPE. If required, sections of these parts, for example, the needle 35 with regard to the rest of the needle unit 12 or the piston 7 with regard to the rest of the piston body 5, can be made of different materials that, for example, have been manufactured through a two-component injection moulded process.

The assembly of the syringe 1 can take place as follows:
The assembly of needle unit 12 and breaking body 14 is placed on a pressure tool (not shown) and positioned in front of the open proximal end 3b of the syringe body 2. The syringe body 2 with this is already equipped with the integrally formed nozzle seal 30. With this, the pressure tool will only be supported on the needle unit 12 in an axial direction and not on the breaking body 14. By pressing the pressure tool together with the assembly of the needle unit 12 and the breaking body 14 in the syringe body 2 inwardly, first the breaking body 14 will snap shut in the widened part 23 of the syringe body 2. Subsequently, the weakened wall sections 13 will be pulled broken after which the needle unit 12 will be pressed further into the syringe body 2 inwardly until this will also snap shut in the distal end 3a of the syringe body 2. The needle 35 of the needle unit 12 will automatically come to lie, within this context, with its outflow openings 36 in the sealing position against the front part 31 a of the protective cap 31. The pressure tool can then be removed and the syringe 1 can be filled with an injection medium from the proximal end 3b. Next, the piston body 5 can be installed in the syringe body 2 filled with injection medium where the stop cams 10 pass the limiting section 27 of the breaking body 14, fix themselves behind this and automatically activate the piston seal. Additional operations are not required for either activating the piston seal or activating the nozzle seal. They will automatically become operational during the above described assembly.

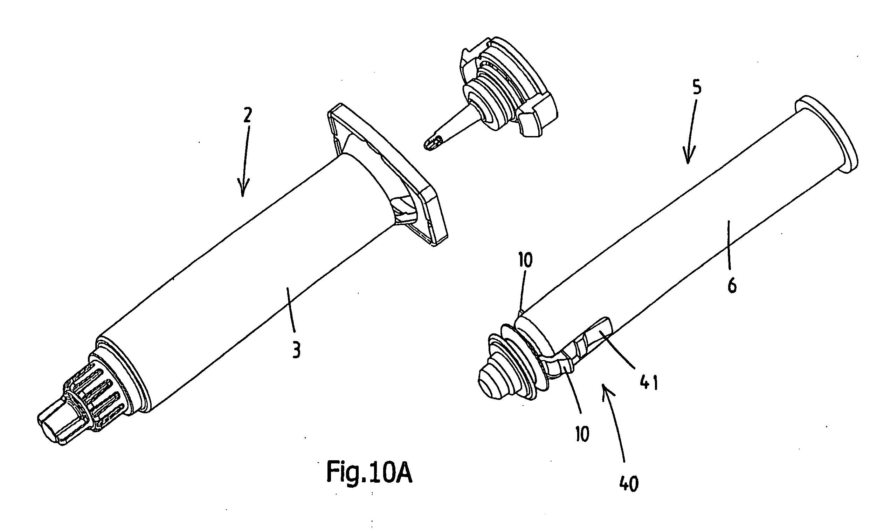

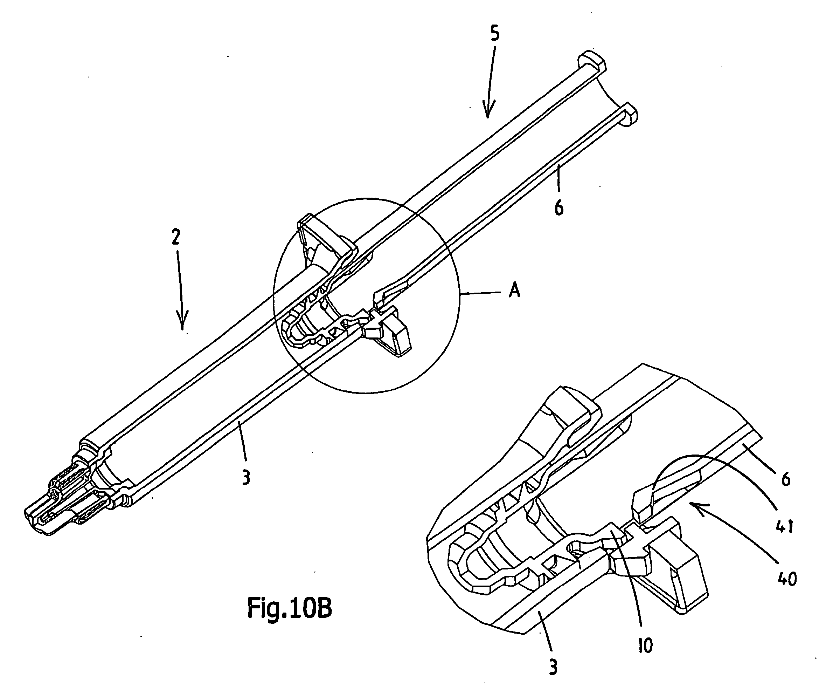

Figure 10 shows a variant in which the piston body 5 is equipped with a pair of protective parts 40 that protrudes inwardly in the radial direction that is formed through protection cams 41 that are outwardly compressable in the radial direction. The protective parts 40 can be found at the backside of the stop cams 10 in the axial direction. The function of the protection parts 40 is to prevent that anybody can put the stop cams 10 in the compressed position, can, subsequently, fix them in this compressed position and, next, can pull the piston body 5 unnoticed from the syringe body 2, without discernibly damaging the piston seal. Putting the stop cams 10 in the compressed position can, for example, take place by pressing the piston body 5 somewhat further into the piston body 2 after assembly. The stop cams 10 are then inwardly pressed by the internal perimeter wall of the cylindrical part 3. Subsequently, someone could try to stick something sharp into the piston rod 6 that can grab on to the compressed stop cams 10 in order to block this in the compressed position. The protection parts 40 block this because they form a screen for the stop cams 10 towards the back. By manufacturing the protection parts 40 in an outwardly compressable manner in the radial direction they will not be in the way of an integral forming thereof on the piston body, for example, through an injection moulding process. A mould core can then be pulled out by compressing the protection parts 40. In a variant a different type of blocking mechanism can be provided between the thumb support 8 and the stop cams 10 instead of the compressable protection parts 40, for example, by having the cylindrical part 6 terminate just before the stop cams 10 with a fully or partially closed bottom plate.

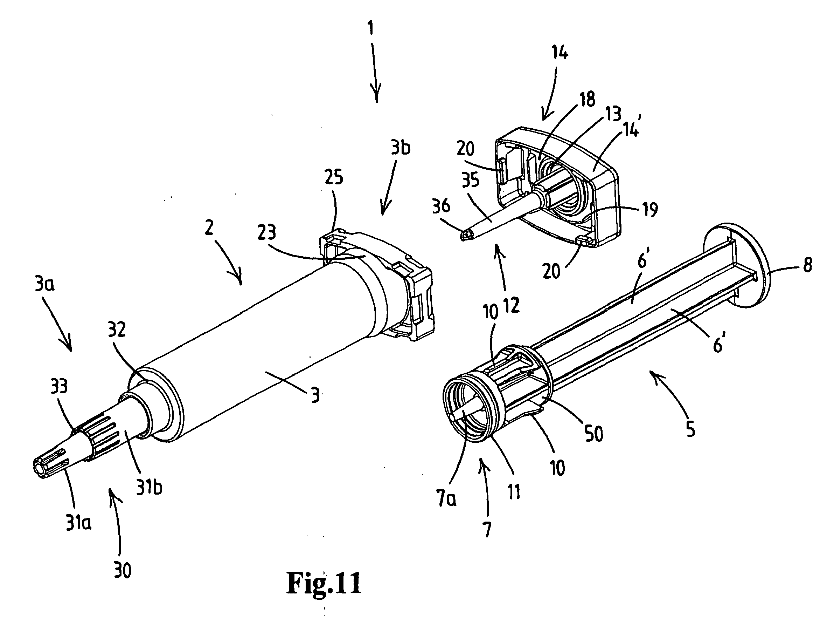

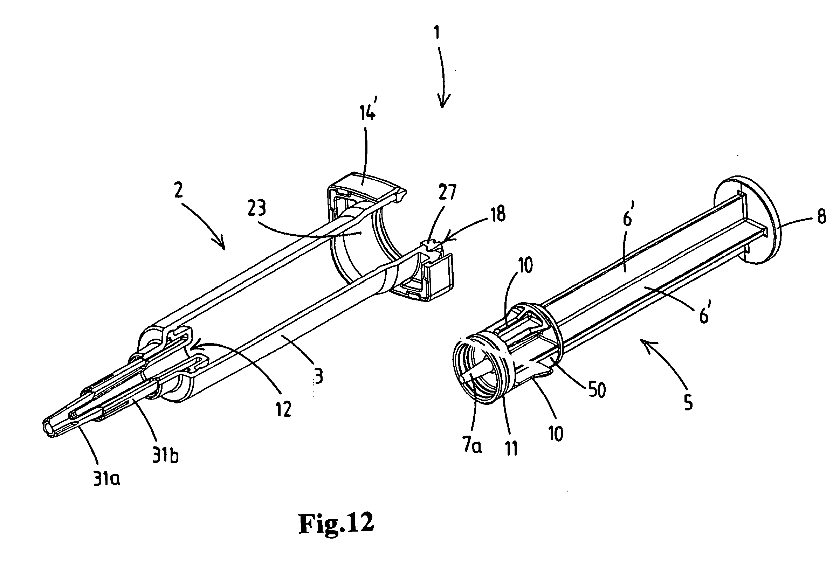

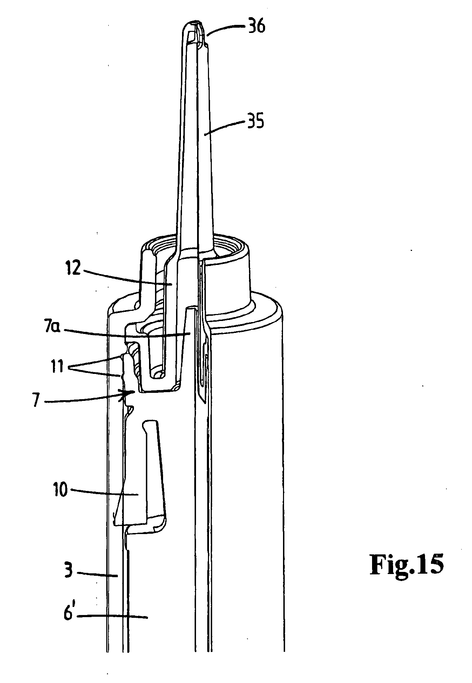

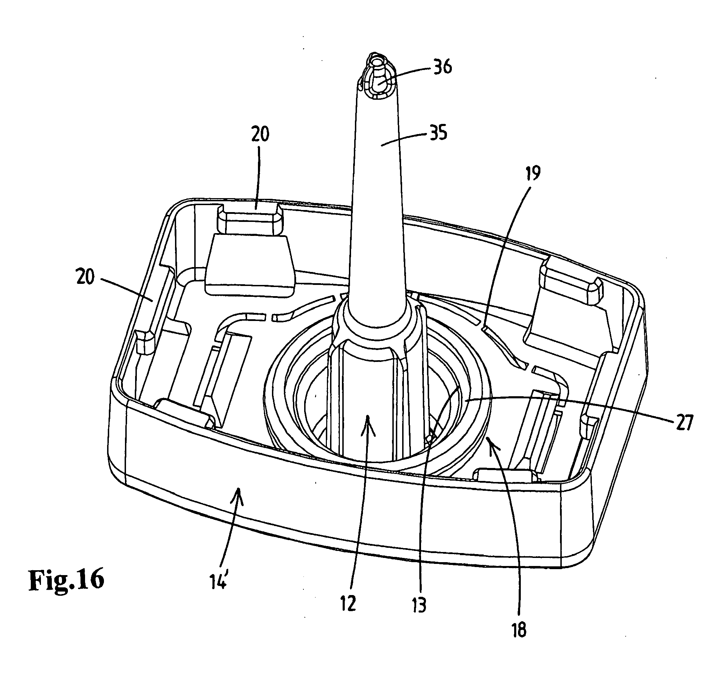

A variant is shown in figures 11-16 where similar parts are indicated with the same reference numbers. Instead of a cylindrical piston rod, the piston rod 6 is now executed with a cruciform cross section with two wall sections 6' that extend in the axial direction that are at right angles with regard to each other. Two integrally formed diametrically opposite stop cams 10 have also been provided here that have now been partially integrated into the wall sections 6'. The stop cams 10 extend, as it where, into recesses in the wall sections 6' and due to this, again have the freedom to inwardly compress in the radial direction. The piston 7 is also an integral part of the piston body 5 here and is equipped with an elastically ductile ring-shaped wall section 11. A cone-shaped pin 7a extends in the centre of the piston 7 that partially grips into the needle unit 12 in the slid in position as shown in figure 15. In addition, the piston rod between the stop cams 10 and the thumb support 8 has been provided with a protection part that has here been executed as a circular disc 50. The disc 50 forms a blockade because this mainly seals off the whole of the cross section of the cylindrical part 3 of the syringe body 2 and has, therefore, the same function as the protection parts 40 shown in figure 10, that is, preventing someone from trying to fix the stop cams 10 in their compressed position.

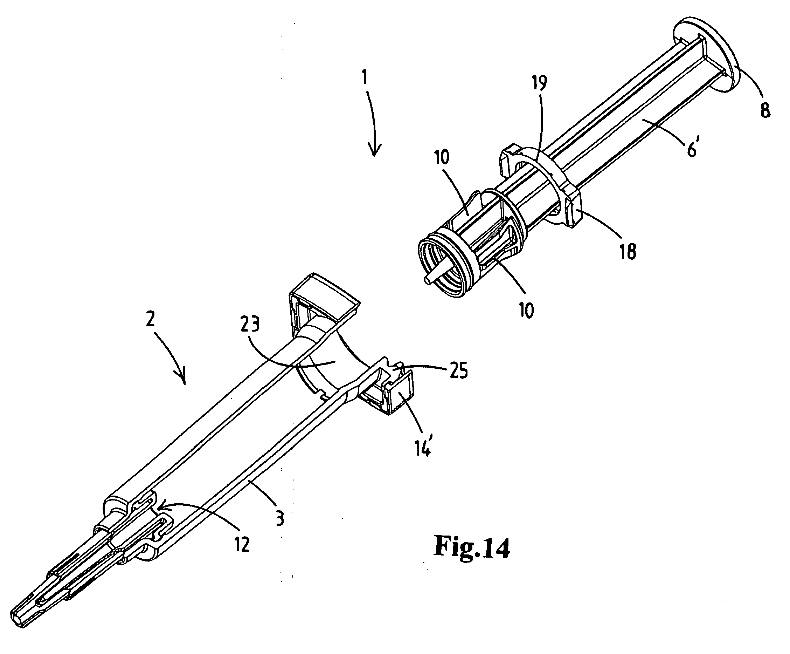

Another difference with the embodiment of figure 1-6 is the construction of the breaking body 14. The breaking body here comprises a mounting section 14' that is set up to grip around the finger grips 25 in the mounted position with mounting bosses 20 in such a way that they cannot be separated. The breaking body 14 also comprises a ring-shaped breaking-out segment 18 linked to the mounting section 14' through weakened wall sections 19. The breaking-out segment 18 comprises the limiting section 27 inwardly protruding in the radial direction. The parts have been constructed in such a way that the radial external dimensions Rsc of the stop cams 10 are greater than the radial internal dimensions Rls of the limiting section 27 and that both Rsc and Rls, in turn, are again greater than the radial external dimensions Rp of the piston 7 and the radial internal dimensions Rcp of the cylindrical part 3 of the syringe body 2, respectively. The breaking-out segment 18 is, moreover, provided as one piece and will, as a result thereof, stay around the piston rod 6 once it has broken away from the mounting section 14' (see figure 14).

Before assembly, or rather the situation as shown in figure 11, the needle unit 12 will be connected to the breaking body 14 through a weakened wall section 13. With this the weakened wall section 13 fully extends as a film connection around the needle unit 12. This has the advantage that no sharp breaking points are created when the wall section 13 is broken, which could otherwise lead to scratches in the syringe body when the needle unit 12 is inserted.

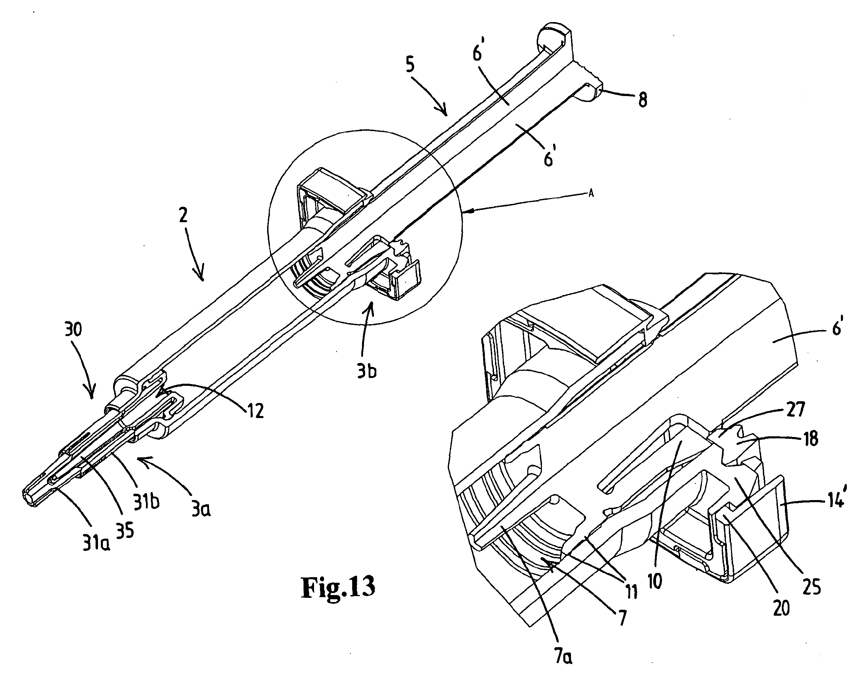

The stop cams 10 will be in the widened part 23 of the syringe body 2 (see figure 13) in the fully assembled and filled state of the syringe 1. This has the advantage that the stop cams 10 can find themselves in their unloaded uncompressed position and, therefore, the plastic will not undergo relaxation. If it is required that syringes are supplied that are only partially filled, then piston bodies can advantageously be applied where the stop cams are positioned in the direction of the thumb support 8 to a lesser or greater extent depending on the contemplated filling level.

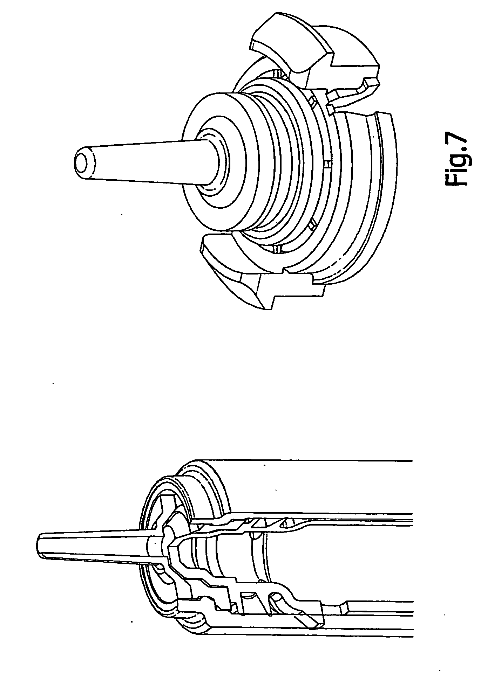

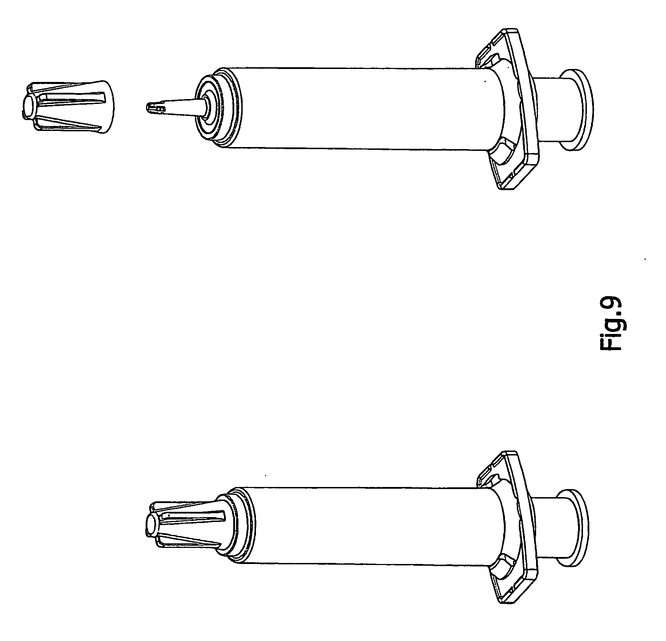

Many variants are possible in addition to the shown embodiments. For example, various parts of the syringe can be given different forms and dimensions. The breaking body can also be provided outside the syringe body instead of in a widened part of the syringe body. Another tamper evident construction can be applied at the distal end instead of the integrally formed nozzle seal in order to reliably seal the distal end. In this case, it is also possible to integrally form a needle unit on the syringe body and/or to apply a needle unit that can be connected to the distal end of the syringe body from the outside. Other types or forms of breaking walls can also be applied as weakened wall sections for the seals that can or cannot be integrally formed. It is even possible to not provide any weakened wall sections. The limiting section will then form, as it where, an unbreakable limit for a piston body that has been stuck passed this with its stop cams. Various variants can also be applied as a needle of which one is shown in figure 7 having only one outflow opening on its free end. A metal needle can also be applied with one or more outflow openings that may or may not flow out laterally. A one-piece protective cap can also be used instead of a two-piece nozzle seal as shown in figure 9.

Thus, a syringe is provided constructed from a minimum number of parts in accordance with the invention that can be easily manufactured and assembled and where the seals are not in the way of the assembly and are automatically activated as a direct result of this assembly. The syringe can be inexpensively manufactured and filled with injection medium during the assembly process of the various parts. The syringe can be effectively used for injecting an injection medium into an animal, into a teat of an udder of a milking animal such as a cow or goat, for example, an ointment with antibiotic properties and/or a hardening medium that can temporarily seal the teat.

## Claims

1. Syringe comprising:
- a syringe body (2) with a cylindrical part (3) that has a nozzle on its distal end (3a);
- a piston body (5) with a piston rod (6) and a piston (7) that closes in a sealing manner the syringe body (2) on its proximal end (3b) and which is slidable in the axial direction of the syringe body (2); and
- a piston seal that limits a movement of the piston body (5) in proximal direction with regard to the cylindrical part (3) and which piston seal has been arranged in such a way that this discernibly damages if the piston body (5) is moved in the proximal direction with regard to the syringe body (2);
in which at least one stop section that protrudes outwardly in the radial direction has been provided on the piston body (5) which stop section in a sealed state is limited in the axial direction by at least one limiting section (27) of the piston seal which is inwardly extending in the radial direction;
**characterized in that**
the stop section protruding outwardly in the radial direction is formed through a stop cam (10) that is inwardly compressable in the radial direction;
in which the limiting section (27) has radial internal dimensions that are greater than the radial external dimensions of the piston (7) and the radial internal dimensions of the cylindrical part (3) of the syringe body (2), respectively, in which the stop cam (10) has radial external dimensions that in a non-compressed position are greater than the radial internal dimensions of the limiting section (27).

2. Syringe in accordance with claim 1, wherein the limiting section (27) of the piston seal comprises a breaking body (14) that is affixed to the proximal end (3b) of the syringe body (2).

3. Syringe in accordance with claim 2, wherein the breaking body (14) comprises at least one breaking-out segment (18) that is connected to another part of the breaking body (14) through at least one weakened wall section (19).

4. Syringe in accordance with claim 2 or 3, wherein the breaking body (14) is fixed to the proximal end (3b) of the syringe body (2) through a snap connection.

5. Syringe in accordance with one of the previous claims, wherein the limiting section (27) is provided in a widened part (23) of the proximal end (3b) of the piston body (2).

6. Syringe in accordance with one of the previous claims, wherein the stop cam (10) which is inwardly compressable in the radial direction is an integral part of the piston body (5).

7. Syringe in accordance with claim 6, wherein the piston rod (6) comprises a wall section in which the stop cam (10) which is inwardly compressable in the radial direction has been integrated.

8. Syringe in accordance with one of the previous claims, wherein the nozzle comprises a needle unit (12) that has been inserted into the syringe body (2) from the proximal end (3b) and is limited by the syringe body (2) in the direction of the distal end (3a).

9. Syringe in accordance with claims 2 and 8, wherein the breaking body (14) and the needle unit (12) are interlinked before mounting in the syringe body (2) through at least one weakened wall section (13), which weakened wall section (13) is broken as a result of an axial insertion force during mounting in the syringe body (2).

10. Syringe in accordance with one of the previous claims, wherein a nozzle seal (30) has been provided on the distal end (3a) that seals the nozzle and which nozzle seal (30) has been constructed in such a way that this will discernibly damage if an outflow opening (36) of the nozzle is released.

11. Syringe in accordance with claim 10, wherein the nozzle seal (30) comprises a protective cap (31) that at least screens off the outflow opening (36) of the nozzle and that is integrally formed on the distal end (3a) of the syringe body (2) through at least one weakened wall section (32).

12. Syringe in accordance with claim 11, wherein the protective cap (31) rests in a sealing manner against the outflow opening (36) of the nozzle.

13. Syringe in accordance with one of the claims 11-12, wherein the protective cap (31) comprises at least two parts (31 a, 31 b) that are interlinked through at least one weakened wall section (33) and that each screen off a successive longitudinal part of the nozzle.

14. Syringe in accordance with one of the previous claims, wherein the breaking body (14) and/or the needle unit (12) are manufactured from plastic, in particular, PE, PP and/or TPE.

15. Syringe in accordance with one of the previous claims, wherein the piston body (5) has been provided with at least one protective part (40) that screens off the stop cam (10) towards the back in the axial direction.

16. Syringe in accordance with one of the previous claims filled with injection medium.

## Patentansprüche

1. Spritze, umfassend:
- einen Spritzenkörper (2) mit einem zylindrischen Teil (3), das an seinem distalen Ende (3a) eine Mündung hat;
- einen Kolbenkörper (5) mit einem Kolbenstab (6) und einem Kolben (7), der auf eine abdichtende Art und Weise den Spritzenkörper (2) an seinem proximalen Ende (3b) schließt und der in der axialen Richtung des Spritzenkörpers (2) verrutschbar ist; und
- eine Kolbendichtung, die eine Bewegung des Kolbenkörpers (5) in proximaler Richtung in Bezug auf das zylindrische Teil (3) begrenzt und wobei die Kolbendichtung derart eingerichtet wurde, dass diese erkennbar beschädigt wird, wenn der Kolbenkörper (5) in der proximalen Richtung in Bezug auf den Spritzenkörper (2) bewegt wird;
bei welcher mindestens ein Stoppabschnitt, der nach außen in der radialen Richtung hervorsteht, an dem Kolbenkörper (5) bereitgestellt worden ist, wobei dieser Stoppabschnitt in einem abgedichteten Zustand in der axialen Richtung durch mindestens einen Begrenzungsabschnitt (27) der Kolbendichtung begrenzt ist, der sich in der radialen Richtung nach innen erstreckt;
**dadurch gekennzeichnet, dass**
der Stoppabschnitt, der nach außen in der radialen Richtung hervorsteht, durch eine Stoppnocke (10) ausgebildet ist die in der radialen Richtung nach innen komprimierbar ist;
bei welcher der Begrenzungsabschnitt (27) radiale interne Abmessungen hat, die jeweils größer als die radialen externen Abmessungen des Kolbens (7) und die radialen internen Abmessungen des zylindrischen Teils (3) des Spritzenkörpers (2) sind, bei welcher die Stoppnocke (10) radiale externe Abmessungen hat, die in einer nicht komprimierten Position größer als die radialen internen Abmessungen des Begrenzungsabschnitts (27) sind.

2. Spritze gemäss Anspruch 1, wobei der Begrenzungsabschnitt (27) der Kolbendichtung einen Bruchkörper (14) umfasst, der an dem proximalen Ende (3b) des Spritzenkörpers (2) angebracht ist.

3. Spritze gemäss Anspruch 2, wobei der Bruchkörper (14) mindestens ein freibrechendes Segment (18) umfasst, das mit einem anderen Teil des Bruchkörpers (14) durch mindestens einen verjüngten Wandabschnitt (19) verbunden ist.

4. Spritze gemäss Anspruch 2 oder 3, wobei der Bruchkörper (14) an dem proximalen Ende (3b) des Spritzenkörpers (2) durch eine Schnappverbindung angebracht ist.

5. Spritze gemäss einem der voranstehenden Ansprüche, wobei der Begrenzungsabschnitt (27) in einem aufgeweiteten Teil (23) des proximalen Endes (3b) des Kolbenkörpers (2) bereitgestellt ist.

6. Spritze gemäss einem der voranstehenden Ansprüche, wobei die Stoppnocke (10), die in der radialen Richtung nach innen komprimierbar ist, ein integrales Teil des Kolbenkörpers (5) ist.

7. Spritze gemäss Anspruch 6, wobei der Kolbenstab (6) einen Wandabschnitt umfasst, in dem die Stoppnocke (10), die in der radialen Richtung nach innen komprimierbar ist, integriert worden ist.

8. Spritze gemäss einem der voranstehenden Ansprüche, wobei die Mündung eine Nadeleinheit (12) umfasst, die in den Spritzenkörper (2) von dem proximalen Ende (3b) eingeführt wurde und die durch den Spritzenkörper (2) in der Richtung des distalen Endes (3a) begrenzt ist.

9. Spritze gemäss den Ansprüchen 2 und 8, wobei der Bruchkörper (14) und die Nadeleinheit (12) vor Befestigung in dem Spritzenkörper (2) durch den mindestens einen verjüngten Wandabschnitt (13) miteinander verknüpft sind, wobei dieser verjüngte Wandabschnitt (13) als Ergebnis einer axialen Einführungskraft während Befestigung in dem Spritzenkörper (2) bricht.

10. Spritze gemäss einem der voranstehenden Ansprüche, wobei eine Mündungsdichtung (30) an dem distalen Ende (3a) bereitgestellt worden ist, das die Mündung abdichtet, wobei diese Mündungsdichtung (30) auf eine solche Art und Weise konstruiert worden ist, dass sie erkennbar beschädigt wird, wenn eine Ausflussöffnung (36) der Mündung freigesetzt ist.

11. Spritze gemäss Anspruch 10, wobei die Mündungsdichtung (30) eine Schutzkappe (31) umfasst, die zumindest die Ausflussöffnung (36) der Mündung abdeckt und die an dem distalen Ende (3a) des Spritzenkörpers (2) durch mindestens einen verjüngten Wandabschnitt (32) integral ausgebildet ist.

12. Spritze gemäss Anspruch 11, wobei die Schutzkappe (31) auf eine dichtende Art und Weise an der Ausflussöffnung (36) der Mündung aufsitzt.

13. Spritze gemäss einem der Ansprüche 11-12, wobei die Schutzkappe (31) mindestens zwei Teile (31a, 31b) umfasst, die durch mindestens einen verjüngten Wandabschnitt (33) miteinander verknüpft sind und die jeweils einen darauffolgenden Längsteil der Mündung abdecken.

14. Spritze gemäss einem der voranstehenden Ansprüche, wobei der Bruchkörper (14) und/oder die Nadeleinheit (12) aus Kunststoff, insbesondere PE, PP und/oder TPE hergestellt sind.

15. Spritze gemäss einem der voranstehenden Ansprüche, wobei der Kolbenkörper (5) mit mindestens einem Schutzteil (40) bereitgestellt worden ist, der die Stoppnocke (10) hin zu der Rückseite in der axialen Richtung abdeckt.

16. Spritze gemäss einem der voranstehenden Ansprüche, die mit einem Injektionsmedium gefüllt ist.

## Revendications

1. Seringue comprenant :
- un corps de seringue (2) avec une partie cylindrique (3) qui présente une buse sur son extrémité distale (3a) ;
- un corps de piston (5) avec une tige de piston (6) et un piston (7) qui ferme de manière étanche le corps de seringue (2) sur son extrémité proximale (3b) et qui est coulissant dans la direction axiale du corps de seringue (2) ; et
- un joint de piston qui limite un mouvement du corps de piston (5) dans la direction proximale par rapport à la partie cylindrique (3) et lequel joint de piston a été disposé de manière à être sensiblement endommagé si le corps de piston (5) est déplacé dans la direction proximale par rapport au corps de seringue (2) ;
dans laquelle au moins une section d'arrêt qui fait saillie vers l'extérieur dans la direction radiale a été prévue sur le corps de piston (5), laquelle section d'arrêt dans un état étanche est limitée dans la direction axiale par au moins une section limite (27) du joint de piston qui s'étend vers l'intérieur dans la direction radiale;
**caractérisée en ce que**
la section d'arrêt faisant saillie vers l'extérieur dans la direction radiale est formée par une came d'arrêt (10) qui est compressible vers l'intérieur dans la direction radiale;
dans laquelle la section limite (27) présente des dimensions internes radiales qui sont supérieures respectivement aux dimensions externes radiales du piston (7) et aux dimensions internes radiales de la partie cylindrique (3) du corps de seringue (2), dans laquelle la came d'arrêt (10) présente des dimensions externes radiales qui, dans une position non comprimée, sont supérieures aux dimensions internes radiales de la section limite (27).

2. Seringue selon la revendication 1, dans laquelle la section limite (27) du joint de piston comprend un corps de rupture (14) qui est fixé sur l'extrémité proximale (3b) du corps de seringue (2).

3. Seringue selon la revendication 2, dans laquelle le corps de rupture (14) comprend au moins un segment d'arrachement (18) qui est relié à une autre partie du corps de rupture (14) par au moins une section de paroi affaiblie (19).

4. Seringue selon la revendication 2 ou 3, dans laquelle le corps de rupture (14) est fixé à l'extrémité proximale (3b) du corps de seringue (2) par un assemblage à enclenchement.

5. Seringue selon l'une quelconque des revendications précédentes, dans laquelle la section limite (27) est prévue dans une partie élargie (23) de l'extrémité proximale (3b) du corps de seringue (2).

6. Seringue selon l'une quelconque des revendications précédentes, dans laquelle la came d'arrêt (10) qui est compressible vers l'intérieur dans la direction radiale fait partie intégrante du corps de piston (5).

7. Seringue selon la revendication 6, dans laquelle la tige de piston (6) comprend une section de paroi, dans laquelle a été intégrée la came d'arrêt (10) qui est compressible vers l'intérieur dans la direction radiale.

8. Seringue selon l'une quelconque des revendications précédentes, dans laquelle la buse comprend une unité d'aiguille (12) qui a été insérée dans le corps de seringue (2) depuis l'extrémité proximale (3b) et est limitée par le corps de seringue (2) dans la direction de l'extrémité distale (3a).

9. Seringue selon les revendications 2 et 8, dans laquelle le corps de rupture (14) et l'unité d'aiguille (12) sont intégrés avant le montage dans le corps de seringue (2) par au moins une section de paroi affaiblie (13) qui est rompue en raison d'une force d'insertion axiale pendant le montage dans le corps de seringue (2).

10. Seringue selon l'une quelconque des revendications précédentes, dans laquelle un joint de buse (30) a été prévu sur l'extrémité distale (3a) qui rend étanche la buse et lequel joint de buse (30) a été construit de manière à être endommagé de manière visible si une ouverture de sortie (36) de la buse est libérée.

11. Seringue selon la revendication 10, dans laquelle le joint de buse (30) comprend un capuchon protecteur (31) qui obture au moins l'ouverture de sortie (36) de la buse et qui est intégralement formé sur l'extrémité distale (3a) du corps de seringue (2) par au moins une section de paroi affaiblie (32).

12. Seringue selon la revendication 11, dans laquelle le capuchon protecteur (31) repose de manière étanche contre l'ouverture de sortie (36) de la buse.

13. Seringue selon l'une quelconque des revendications 11 à 12, dans laquelle le capuchon protecteur (31) comprend au moins deux parties (31a, 31b) qui sont reliées par au moins une section de paroi affaiblie (33) et qui obturent chacune une partie longitudinale successive de la buse.

14. Seringue selon l'une quelconque des revendications précédentes, dans laquelle le corps de rupture (14) et/ou l'unité d'aiguille (12) sont fabriqués en plastique, en particulier en PE, PP et/ou TPE.

15. Seringue selon l'une quelconque des revendications précédentes, dans laquelle le corps de piston (5) a été doté d'au moins une partie protectrice (40) qui obture la came d'arrêt (10) vers l'arrière dans la direction axiale.

16. Seringue selon l'une quelconque des revendications précédentes remplie d'un produit d'injection.
